# FASCICULE DE BREVET EUROPEEN

(11) **EP 0 815 445 B1**
(45) Date de publication et mention de la délivrance du brevet: **06.10.1999**
(21) Numéro de dépôt: 96908159.5
(22) Date de dépôt: 20.03.1996
(51) Int. Cl.: G01N 33/18

(54) **PROCEDE ET DISPOSITIF POUR REALISER DES PROFILS VERTICAUX DE MESURES DE GRANDEURS CARACTERISTIQUES D'UNE MASSE D'EAU MARINE**
VERFAHREN UND VORRICHTUNG ZUM ERZEUGEN VON VERTIKALEN MESSPROFILEN VON MEERWASSERCHARAKTERISTISCHEN GRÖSSEN
METHOD AND DEVICE FOR PRODUCING VERTICAL PROFILES OF MEASURED VARIABLES CHARACTERISING A BODY OF SEA WATER

(30) Priorité: 21.03.1995 FR 9503269; 03.11.1995 FR 9512996
(43) Date de publication de la demande: 07.01.1998
(73) Titulaire: SOCIETE BRETONNE D'INSTRUMENTATION OCEANOGRAPHIQUE BRIO, 29900 Concarneau (FR)
(72) Inventeur: GUINARD, Jean-Paul, F-91000 Evry (FR)
(74) Mandataire: Colas, Jean-Pierre
(86) Numéro de dépôt international: FR9600418
(87) Numéro de publication internationale: WO9629597

(56) Documents cités:
- WO-A-93/17334
- DATABASE WPI Week 8850 Derwent Publications Ltd., London, GB; AN 88-359554 [50] XP002009882 & SU,A,1 401 331 (OCEANOLOGY INST) , 7 Juin 1988

## Description

### Domaine technique de l'invention

La présente invention est relative à un procédé et à un dispositif pour réaliser des profils verticaux de mesures de grandeurs caractéristiques d'une masse d'eau marine et, plus particulièrement, à un tel procédé et un tel dispositif faisant appel à des sondes plus légères que l'eau équipées de capteurs de mesure sensibles aux dites grandeurs.

### Arrière-clan de l'invention

L'observation de la structure, des mouvements et de la composition chimique et biologique des couches d'eau marines a commencé au XIXème siècle, pour un but de connaissance fondamentale; les besoins d'observation ont connu une importante accélération pendant la seconde guerre mondiale et depuis la fin de celle-ci, par suite du développement des moyens de guerre sous-marine, pour les tranches d'eau comprises entre 0 et -1000m.; au cours des vingt dernières années, la dégradation de l'environnement terrestre ("effet de serre", évolution du climat, pollutions...), et la mise en évidence de l'importance du rôle des océans dans les échanges climatiques et chimiques avec l'atmosphère, a généré de nouveaux besoins d'observation de l'océan, aux plans qualitatif (présentation des résultats pour assimilation facile par ordinateur, observation "opérationnelle") et quantitatif (répartition géographique, rythme, densité, profondeur des observations). Les dirigeants des nations de la planète ont manifesté leur intérêt pour ces questions en particulier au cours de la Conférence mondiale sur l'environnement de RIO en 1992, qui s'est tenue sous l'égide de l'ONU; ils ont décidé de mettre en commun les résultats des recherches, et de coordonner les efforts des nations en vue de la création d'un "Système d'Observation de l'Océan Mondial" (En anglais, Global Ocean Observing System, GOOS). Enfin, les progrès considérables apportés à l'observation des Océans par les satellites (observation permanente, à haute résolution, sur l'ensemble des mers du Globe), d'une part, le développement des techniques de modélisation numérique des mouvements de fluides d'autre part, ont apporté de nouveaux outils pour traiter les problèmes de la description et de la prévision des mouvements de l'Océan. Toutefois, les techniques d'observation par satellites ne sont pas suffisantes pour traiter l'ensemble du problème: par nature limitées à l'observation des couches d'eau superficielles, elles n'ont pas accès à la mesure des grandeurs caractéristiques des couches profondes; or la recherche moderne a mis en évidence l'importance de ces dernières, en particulier de la circulation, pour les échanges d'énergie, de matières, et la production végétale et animale: le système d'observation mondial ne peut se passer d'un important segment dit in *situ.* D'importants efforts ont été déployés pour recenser les procédés et dispositifs existants, et évaluer leur capacité à répondre aux nouveaux besoins (cf. par exemple le rapport du Groupe d'experts internationaux réalisé à la demande de l'UNESCO (Paris, 30 Mars au 2 Avril 1993), intitulé: "Global Ocean Systems Dynamics, Globec Report n°3: Sampling and Observational Systems", Ed. Ms. Amy Freise, Executive Secretary, GLOBEC INTERNATIONAL, Chesapeake Biological Laboratory, Post Office Box 38, Solomons, Maryland, 20688 USA). Aucun de ces procédés et dispositifs existants n'apparaît bien adapté aux tâches nouvelles, comme nous le montrerons dans ce qui suit.

Le procédé le plus ancien pour réaliser des profils de mesure de grandeurs caractéristiques d'une masse d'eau est de prélever des échantillons à plusieurs paliers de profondeur, par "bouteilles" descendues au bout d'une élingue, et de les analyser ensuite au laboratoire (Procédé dit "hydrologique"). Ce procédé présente l'avantage de permettre des mesures très précises de nombreuses grandeurs, en particulier chimiques et biologiques. Pour cette raison, il continue d'être utilisé couramment dans un but de connaissance scientifique. Par contre, au regard des besoins cités ci-dessus, il présente les inconvénients suivants: (a) son coût est très élevé (il ne peut être mis en oeuvre que par navire hydrographique spécialisé, stoppé pendant le temps du prélèvement, qui est long, compte tenu de la lourdeur du matériel; il nécessite la présence de personnel spécialisé et de moyens de laboratoire à bord; il ne peut être mis en oeuvre par mauvais temps...), (b) l'échantillonnage qu'il procure ne donne qu'une très mauvaise résolution tant au plan spatial que temporel (il est pratiquement impossible de suivre les mouvements d'une masse d'eau par ce procédé) (c) le processus d'acquisition des résultats est peu automatisable et se prête mal à des applications de surveillance.

On connaît également d'autres procédés et dispositifs réalisant une (ou des) mesure (s) directe (s) par capteur (s) porté(s) par la sonde de la (ou des) grandeur(s) à mesurer, poursuivant certains buts de l'invention, mais fonctionnant selon des principes différents. Ils peuvent être classés en quatre familles principales, selon que le déplacement de la sonde en immersion est obtenu: (A) par filage d'un câble à partir d'un navire (dans ce cas, on utilise en général le câble comme support de transmission des données mesurées vers le navire; les mesures sont enregistrées à bord de celui-ci), (B) par largage à partir d'un navire, la sonde étant entraînée par son poids (dans ce cas, les valeurs mesurées sont en général transmises à bord du navire en temps réel par voie filaire), (C) par filages et remontées successifs le long de la ligne de mouillage d'une bouée ancrée (dans ce cas, on recueille les données au niveau de la bouée, on les transmet à un enregistreur situé dans le corps de la bouée, pour retransmission ultérieure à un satellite de collection de données), et enfin (D) en utilisant les mouvements verticaux d'une bouée dérivante à immersion commandable (dans ce cas, les valeurs mesurées sont enregistrées à bord de la bouée pour retransmission au centre de traitement soit en temps peu différé par voie acoustique, soit de temps en temps, à l'occasion de remontées en surface, par voie radioélectrique vers un satellite de collection de données).

On peut citer, à titre d'exemples, les descriptions ou réalisations suivantes: pour le cas (A), l'appareil connu sous le nom d'enregistreur bathythermographique, perfectionné constamment, en particulier pour permettre le sondage à partir d'un navire en route (cf. J.G. Dussereault et R.A. Clarke, Bedford Institution of Oceanography, Canada, "A system to collect température and salinity profiles from vessel underway" in Proceedings of the OCEANS 94 conference, I-402); pour le cas (B), les procédés et dispositifs dits XBT (sondes perdables pour mesures bathythermiques) ou XCTD (sondes perdables pour mesures de conductivité, température et pression), construites en particulier par la Société américaine Sippican Inc. et fréquemment mis en oeuvre par des navires marchands d'"opportunité" sur leur route commerciale (Cf. par exemple brevet US n° 4,854,728 du 8/8/89 ; pour le cas (C), des communications récentes décrivent un dispositif destiné à l'océanographie de surveillance dit VCTD/YOYO; enfin, pour le cas (D), divers dispositifs sont cités, en particulier "RAFOS" (cf. Rossby et al., "The RAFOS System", Jour. of Atmos. and Ocean. Tech., vol.3, N°4 (Dec. 1986), "MARVOR" (cf.: M. Ollitrault et al., "MARVOR, a multi-cycle RAFOS Float" in Sea Technology, Feb. 1994), et enfin "Autonomous Oceanographic Profiler" (cf. le brevet n° WO 93/17334, intitulé "Autonomous oceanographic profiler" de K. McCOY, ainsi que l'article "The autonomous profiling vehicle" du même auteur in Proceedings of the OCEANS 94 Conference, I-419).

Au regard des besoins actuels et futurs de la recherche et de l'observation opérationnelle de l'Océan évoqués ci-dessus, chacune des méthodes décrites se caractérise par des avantages propres, mais aussi des limitations; aucune ne répond aux demandes nouvelles les plus importantes, à savoir d'une part l'observation de l'évolution historique de la composition de l'Océan en des points géographiques donnés, et d'autre part, la constitution de grands systèmes d'observation in situ de l'Océan, à différentes échelles de temps et d'espace, complémentaires des systèmes d'observation satellitaires. Plusieurs auteurs ont constaté par exemple la pauvreté des bases de données de séries chronologiques d'observations (cf. par ex. "Decade-to-Century changes in the Ocean Circulation", Carl WUNSCH, dans la revue "Oceanography", vol. 5, N°2, Dec. 1992, pp. 99-106), pauvreté qui traduit les carences des procédés et dispositifs existants à produire les types d'observation demandés. Les développements les plus récents - en particulier les flotteurs dérivants à immersion commandée - ont permis de mettre en évidence, au cours des vingt dernières années, l'existence et l'importance de la circulation profonde; malheureusement, ils ne sont pas susceptibles de constituer des systèmes d'observation eulériens répondant aux besoins de la modélisation de la circulation océanique, ni de produire des séries chronologiques d'observations en un lieu donné.

Les procédés de type (A), (B) et (C) présentent les limitations suivantes: s'ils sont mis en oeuvre par navires spécialisés, leur coût opératoire est élevé; si au contraire ils sont mis en oeuvre par des navires d'opportunité, leurs possibilités d'échantillonnage sont limitées dans l'espace (y compris en profondeur) et dans le temps (cf. le brevet n° WO 93/17334).

Le procédé de type (D) présente une première limitation : utilisés comme traceurs pour suivre une masse d'eau, les flotteurs dérivants à immersion contrôlée fournissent une information relative au déplacement d'un élément de la masse d'eau, mais ne renseignent pas sur le déplacement de ses voisines (aussi, le type d'information qu'elles fournissent est peu intéressant pour le traitement par modèles numériques).

Tous les systèmes décrits ci-dessus ont l'inconvénient de contraindre très fortement l'expérimentateur dans le choix d'une stratégie d'échantillonnage, tant au niveau des emplacements géographiques des lieux de sondage qu'à celui de la chronologie de ceux-ci. Ces aspects sont importants pour la réalisation de modèles de circulation: avec les procédés actuels, l'expérimentateur doit se satisfaire de points d'entrée dans son modèle situés à des positions géographiques souvent sans rapport avec une optimisaticn de celui-ci, soit pour des raisons de coût, soit à cause du principe du procédé utilisé: c'est ainsi que la couverture de certaines zones critiques - comme par exemple certains détroits éloignés des routes maritimes, particulièrement importante pour l'expérimentation, n'est pas satisfaisante avec les procédés actuels.

Aucun des systèmes actuels ne permet de réaliser les prélèvements de manière simultanée ou coordonnée entre différents sites d'observation, de façon à fournir des "vues" synoptiques d'une situation des masses d'eau, par exemple des coupes horizontales successives réalisées au même instant sur de vastes régions géographiques, ou encore des séries d'observations "phasées" par rapport à un phénomène périodique, comme la marée ou la saison. Or, cette qualité est importante pour optimiser la modélisation et pour interpréter des prévisions réalisées par les modèles de circulation.

Une autre limitation des procédés actuels d'échantillonnage est la suivante: les expérimentateurs sont dans l'obligation d'avoir recours à une multiplicité de sources utilisant des instruments de nature et d'origines diverses, de les rassembler et de les combiner; le rapprochement des résultats provenant de ces diverses sources oblige les expérimentateurs à renforcer leurs procédures et moyens d'étalonnage et d'intercalibration. Ceci est d'autant plus grave que les procédures d'étalonnage de certains instruments sont manuelles.

Une autre limitation concerne les procédés utilisant des bouées dérivantes ou ancrées: certains capteurs, en particulier électriques (par exemple, mesure de conductivité de l'eau par sondes à électrodes) ou optiques (par exemple pour mesures biochimiques), devant séjourner pendant de longues périodes dans le milieu environnant, doivent être protégés contre les agressions de celui-ci, et recalibrés de temps en temps, ce qui rend leur mise en oeuvre difficile.

Une autre limitation concerne tous les systèmes (A), (B) et (D) : elle porte sur la longueur des délais de transmission aux centres de traitement des observations faites (quelques semaines à quelques mois pour les observations à partir de navires, quelques mois pour les flotteurs à immersion programmée). De plus, dans les cas (A) et (B), ces délais sont soumis aux aléas des programmes de navigation des navires supports (changements de programme des navires d'opportunité ou spécialisés, conditions de mer, pannes ou difficultés techniques ...). Cette limitation a des conséquences très dommageables a/ sur le coût et l'efficacité des équipes et des moyens chargés du traitement et de la diffusion des données, et b/ sur le contrôle de qualité des mesures dans le cas d'un système opérationnel. Il est fort pénalisant dans le cas d'expérimentations scientifiques, dont la durée est longue par rapport au rythme des travaux des chercheurs. Enfin, il a été constaté que ces délais ne sont pas négligeables au regard de certains phénomènes à observer, et peuvent donc être à la source d'erreurs importantes.

Une autre limitation concerne principalement les procédés de types (A) et (B) ci-dessus: la mise en oeuvre des instruments, le recueil des données et leur transmission au centre de traitement font souvent appel à des interventions manuelles et à des moyens de transmission multiples; même si les procédures de saisie et de transmission sont parfaitement codifiées, il existe là une source d'incidents de fonctionnement préjudiciables à la fiabilité globale d'exploitation du centre. De plus, on ne peut espérer obtenir d'importantes économies d'échelle lorsque le nombre de sondages augmente. Or, il convient d'offrir de plus en plus aux utilisateurs un service de diffusion de résultats bruts et/ou élaborés diversifié et fiable, et de garantir la confidentialité d'accès à certains utilisateurs de "produits" classifiés.

Une autre limitation concerne les procédés de type (C) et (D) ci-dessus: les bouées utilisées présentent un danger pour la navigation, fonction de leur masse en surface, qui atteint plusieurs dizaines de kg.

La présente invention a pour but de fournir un procédé pour réaliser des profils verticaux de mesures de grandeurs caractéristiques d'une masse d'eau marine , qui ne présente pas les inconvénients ou limitations invoquées ci-dessus, et qui présente en outre divers avantages dont en particulier la possibilité de construire des systèmes d'observation de taille adaptée au champ spatio-temporel concerné, en donnant à l'opérateur du système les moyens de maîtriser directement, sans intermédiaire, toutes les étapes du processus de recueil des informations, depuis l'élaboration du programme de recueil des données jusqu'à la diffusion finale des résultats.

On atteint ce but de l'invention ainsi que d'autres qui apparaîtront à la lecture de la description qui va suivre avec un procédé pour réaliser des profils verticaux de la mesure d'au moins une grandeur caractéristique d'une masse d'eau marine, à l'aide d'au moins une sonde plus légère que l'eau, équipée d'un capteur de mesure de ladite grandeur, ce procédé étant remarquable en ce que a) on coule ladite sonde sur le fond marin par lestage, b) on commande la remontée de la sonde et l'activation du capteur pour l'enregistrement de la mesure de ladite grandeur au long de la colonne d'eau traversée par la sonde jusqu'à la surface de la masse d'eau, et c) on commande une transmission des mesures relevées vers un centre d'exploitation de ces mesures.

Suivant un mode de réalisation préféré du procédé suivant l'invention, on coule une pluralité de sondes sur le fond marin et on commande séquentiellement la remontée de chacune des sondes. En variante, on coule plusieurs groupes de sondes en des sites prédéterminés du fond marin, et on commande la remontée coordonnée d'au moins une pluralité de sondes provenant de groupes différents.

Pour la mise en oeuvre du procédé, l'invention fournit un dispositif comprenant : a) au moins une sonde munie d'un capteur de mesure de la grandeur à relever, de moyens de stockage et de transmission des mesures réalisées et de moyens pour lui donner une flottabilité positive, et b) une base lestée pour reposer sur le fond marin et équipée de moyens pour retenir temporairement ladite sonde et libérer celle-ci à un instant prédéterminé.

D'autres caractéristiques et avantages du procédé suivant l'invention apparaîtront à la lecture de la description qui va suivre et à l'examen du dessin annexé dans lequel:
- la Fig. 1 est une vue schématique, en coupe axiale, du dispositif suivant l'invention,
- la Fig. 2 est une vue schématique, en coupe axiale, d'une sonde faisant partie du dispositif selon l'invention,
- la Fig. 3 est un schéma qui illustre le procédé suivant l'invention,
- la Fig. 4 est un schéma de l'électronique du dispositif selon l'invention,
- la Fig. 5 est un schéma de principe du mécanisme d'éjection des sondes,
- la Fig. 6 est une vue partielle agrandie illustrant le montage d'une sonde sur le dispositif.

### Description de l'invention.

La présente invention porte sur un procédé et un dispositif ayant pour buts principaux a) de réaliser économiquement des séries chronologiques de profils verticaux de mesure de grandeurs caractéristiques d'une colonne d'eau marine en un emplacement géographique donné, b) de constituer l'élément de base d'un système d'observation in situ de l'Océan, capable de réaliser des séries chronologiques d'échantillonnage selon trois dimensions d'une masse d'eau marine, ce système étant lui-même obtenu par la mise en oeuvre coordonnée de plusieurs dispositifs. Une première application de l'invention est décrite: elle concerne les grandeurs "température" et "conductivité" desdites colonnes ou masses d'eau marines, en fonction de la profondeur. Son extension peut être envisagée à d'autres mesures (caractéristiques optiques, fluorescence, composition chimique, etc...).

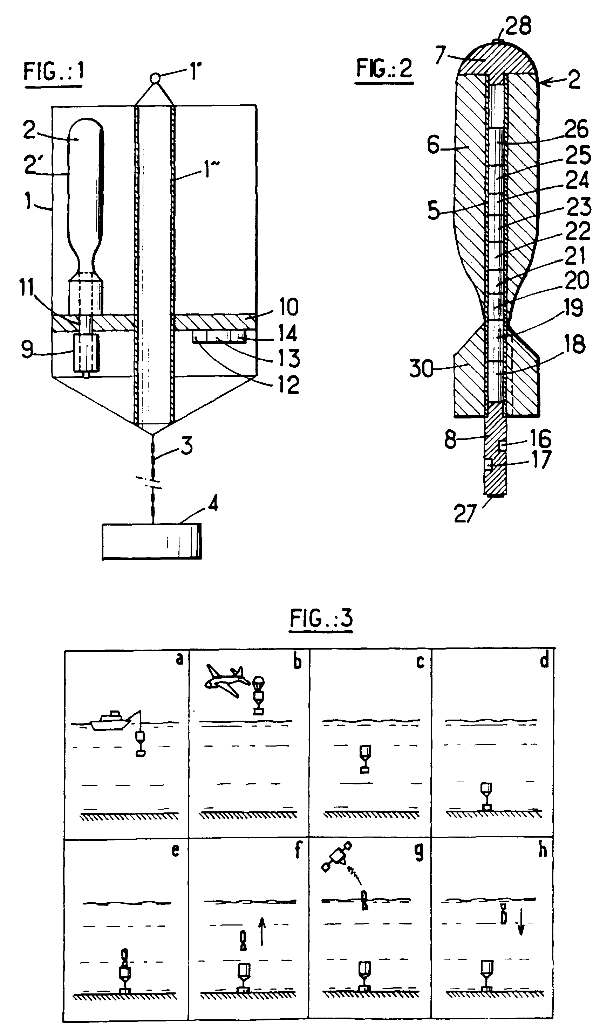

Le dispositif, ou station, suivant l'invention comporte (cf. Fig. 1), une base 1, ayant la forme d'une structure réticulée, de contour extérieur cylindrique, aux parois ouvertes, reliée par un orin 3 à un corps mort 4, une (ou plusieurs) sonde(s) 2 fixées sur la base 1, et un anneau de suspension 1' pouvant recevoir soit un parachute équipé d'un largueur à pression, soit une élingue largable depuis le bord d'un navire. La station est conçue pour être mise à l'eau par aéronef ou navire de surface. L'anneau de suspension 1' est fixé à l'extrémité supérieure d'un tube central 1" faisant partie de la base 1.

La base 1 est composée elle-même d'un châssis 10, qui supporte un ou plusieurs réceptacles 11 conçus pour assurer le maintien mécanique des sondes 2 et contenir des mécanismes d'éjection des sondes. Enfin, un programmateur 12 de commande d'éjection des sondes, un capteur de pression 13 et leur batterie d'alimentation électrique 14 sont fixés sur la base 1.

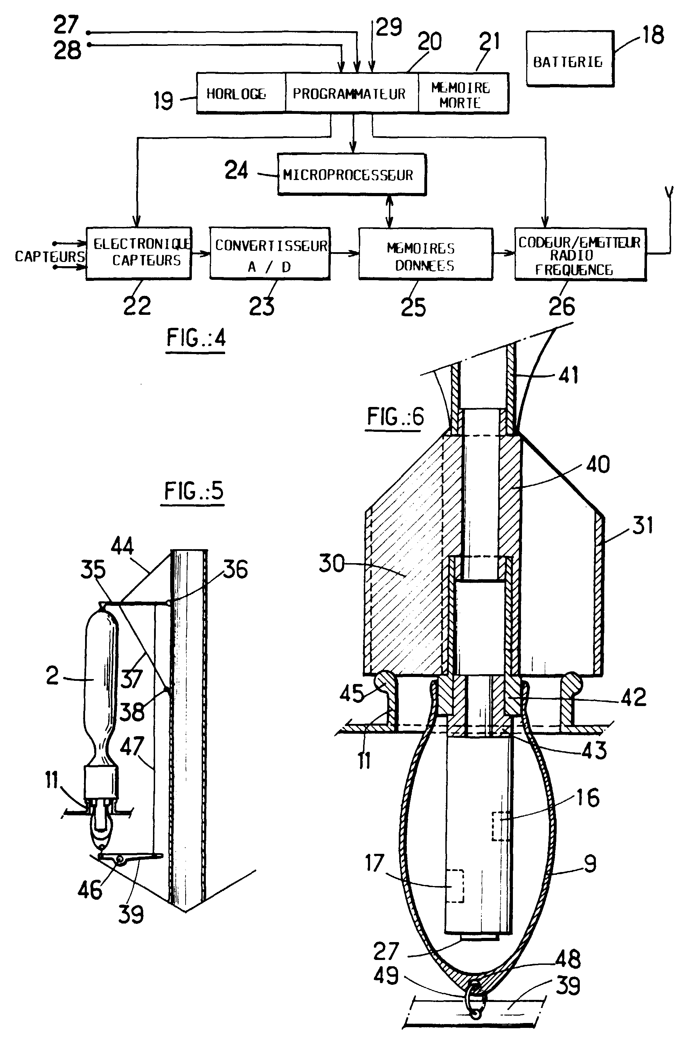

Chaque sonde 2 comprend (Fig.2 et 6) : un corps 5, de forme tubulaire, sur lequel sont fixés : une pièce porte-capteurs 8 conçue pour porter des capteurs 16, .17 et une électrode 27 et obturer une extrémité du corps 5 ; un support d'antenne 7 conçu pour porter une antenne radioélectrique (non représentée) et une électrode 28 et obturer l'autre extrémité du corps 5 ; des empennages 30 réunis par un carénage cylindrique 31; un élément 6 propre à donner une flottabilité positive à l'ensemble de la sonde.

Le corps 5 et les pièces d'extrémité 7 et 8 sont dimensionnés pour résister à la pression d'immersion et maintenir des moyens électroniques contenus dans le corps 5 à la pression atmosphérique. Lesdits moyens électroniques, représentés avec plus de détails à la Fig. 4, comprennent une batterie 18, une horloge 19 et son programmateur 20, au programme enregistré dans une mémoire morte 21, une électronique capteurs 22, un convertisseur analogique/numérique 23, un microprocesseur 24 et sa mémoire de données 25, un codeur émetteur radioélectrique 26.

Les signaux délivrés par les capteurs sont mis en forme et échantillonnés en 22, numérisés et 23 et mis en mémoire en 25. Le programmateur 20 active sélectivement les divers blocs 22 à 26. Le microprocesseur 24 gère l'acquisition, le traitement, la mise en mémoire et le codage des données.

Une entrée de téléchargement 29 permet de charger diverses informations dans l'électronique avant mise en place de la sonde 2 sur la base 1. La masse électrique des circuits électroniques est connectée à une pièce centrale 40 du corps 5, conductrice de l'électricité et solidaire des empennages 30 et du carénage 31. Cette masse assure une bonne prise du potentiel de l'eau environnante, d'une part en utilisant un matériau caractérisé au moins à sa surface par un potentiel d'oxydo-réduction faible (cas du zinc par exemple), d'autre part, grâce à une importante surface de contact avec le milieu. Les empennages 30 et le carénage 31 seront avantageusement réalisés dans ce même matériau conducteur de l'électricité et connectés à la pièce centrale 40 métallique du corps 5.

Le pied de sonde est constitué d'un tube isolant 42 sur lequel la pièce porte-capteurs 8 est emmanchée par l'intermédiaire d'une virole 43. Cette *virole* 43 est réalisée dans un métal différent de celui de la pièce centrale 40, susceptible de s'électrolyser au contact de l'eau de mer en quelques semaines. La virole 43 est reliée électriquement à la pièce centrale 40. Elle reste isolée du milieu marin par la capsule 9 qui s'appuie sur une portée du tube isolant 42 jusqu'au retrait de celle-ci. C'est à partir de ce moment que l'électrolyse se développe: on dispose ainsi d'un moyen simple et efficace d'autodestruction d'un élément de la paroi de la sonde 2 propre à provoquer à terme l'envahissement par l'eau de l'intérieur du corps 5 et la chute de la sonde vers le fond marin (Fig. 3,h).

Les électrodes 27 et 28 sont utilisées pour détecter les instants d'éjection et d'arrivée en surface de la sonde 2, respectivement. Les capteurs 16, de température, et 17, de conductivité, par exemple, sont maintenus immergés dans un liquide, lui-même contenu dans une capsule 9, depuis la fin de montage en usine jusqu'à l'éjection.

Les étapes du procédé pour réaliser des profils verticaux de mesures de grandeurs caractéristiques d'une masse d'eau marine, faisant l'objet de la présente invention sont les suivantes:
- en fin de fabrication en usine, avant de sceller l'ensemble de l'électronique dans le corps 5, on affecte à chaque sonde 2 un numéro d'identification et on le charge dans la mémoire 25 de ladite sonde,
- on étalonne chaque chaîne de mesure de chaque sonde, après nettoyage des capteurs, en ambiance propre, à partir de références; par exemple, pour des mesures de conductivité et de température, les capteurs 16 et 17 correspondants de chaque sonde sont immergés successivement dans plusieurs bains étalonnés en salinité, dont on fait varier la température. On recueille les données d'étalonnage, on les enregistre et on les transmet à un centre de traitement et d'exploitation des mesures, avec le numéro d'identification de la sonde,
- on applique sur les capteurs 16 et 17 la capsule étanche 9 remplie d'un liquide de référence, on commande une première calibration des chaînes de mesure pour contrôle, on met sous tension l'horloge 19 et on scelle le corps 5,
- on stocke les sondes 2 séparément des bases 1, en atelier jusqu'au début de campagne; pendant ce stockage, l'horloge 19 déclenche à intervalles réguliers la calibration des chaînes de mesure et commande le stockage des données recueillies en mémoire 25,
- peu de temps avant le début d'une campagne de mise à l'eau, on prépare les stations en atelier (soit à terre, soit à bord d'un navire), de la manière suivante : on télécharge dans chaque sonde un n° d'identification "station", ainsi qu'un programme de saisie des mesures en profil; on monte les sondes 2 ainsi préparées sur la base 1, on met sous tension et on charge le programme de travail du programmateur 12.
- à son arrivée sur le site prévu pour l'expérimentation, le véhicule porteur manoeuvre pour larguer la station au point prévu, soit par élingage s'il s'agit d'un navire (Fig.3a), soit par parachutage s'il s'agit d'un aéronef (Fig.3b); il relève l'emplacement de mise à l'eau à partir de ses instruments de navigation. Aucun test de fonctionnement n'est nécessaire avant cette opération, qui peut donc être réalisée par le personnel de manoeuvre du bord, sans formation spéciale.
- l'élingue ou le parachute se décrochent sous quelques mètres d'eau sous l'action soit d'un largueur à pression, soit sur commande du navire. La station coule rapidement sous l'effet du poids du lest constitué par le corps mort 4 (Fig.3c) et atteint le fond, en position ancrée sur corps mort, la base 1 et les sondes étant en flottabilité positive (Fig.3d),
- quelques heures après l'arrivée au fond, le programmateur 12 commande la mise sous tension du capteur de pression 13, le chargement de la valeur mesurée dans une sonde à fonction de messager, puis l'éjection de cette sonde; à son arrivée en surface, la sonde transmet les informations recueillies au centre de traitement via un satellite de collection de données,

Pendant le stockage au fond, le programmateur 12 est le seul organe de la base 1 qui reste en permanence sous tension; il commande à des instants préprogrammés l'éjection des sondes 2. Dans une variante du dispositif, qui comporterait un récepteur/décodeur acoustique, l'éjection des sondes pourrait être commandée de l'extérieur par voie acoustique.

De même, dans une sonde, pendant le stockage au fond, l'horloge 19 est le seul organe qui reste alimenté en permanence. Il commande à intervalles réguliers le déclenchement d'une séquence de calibration des chaînes de mesure, et l'enregistrement des résultats correspondants en mémoire.

L'éjection d'une sonde 2 (Fig. 3e) est réalisée par un mécanisme d'éjection sur commande du programmateur 12. Le mécanisme d'éjection sera décrit plus loin en liaison avec les Fig. 5 et 6. En même temps que la libération de la sonde, le mouvement de ce mécanisme permet le dégagement de la capsule 9 du corps de la sonde. Ce dégagement met les capteurs 16 et 17 au contact du milieu, ainsi que l'électrode 27; un circuit électrique se ferme à travers le milieu entre la partie conductrice du corps de sonde et l'électrode 27, ce qui génère un signal qui, détecté par un circuit électronique (non représenté), est ensuite daté et mis en mémoire. L'éjection commande également la mise sous tension de l'électrode 28 et l'établissement d'un courant électrique, par l'intermédiaire du milieu, entre celle-ci et la partie conductrice du corps 5 de la sonde.
- la sonde commence ensuite son ascension, sous l'effet de la poussée d'Archimède, à une vitesse sensiblement constante. Pendant cette ascension, l'horloge 19 commande une suite de séquences de mesure, acquisition, numérisation et enregistrement des données en mémoire de données 25. La chronologie de la suite est celle du programme chargé au cours des opérations de préparation de campagne,
- à l'arrivée en surface, le circuit électrique établi entre l'électrode 28 et la partie conductrice du corps 5 s'interrompt : cet événement est détecté, daté et mis en mémoire selon la même procédure que pour l'éjection; l'instant d'arrivée en surface est ainsi enregistré,
- si la quantité d'informations à transmettre au satellite de collection de données est importante, entraînant par exemple un temps de transmission excessif, on réalise une compression des données enregistrées; la détection de l'arrivée en surface commande alors l'initialisation du traitement correspondant,
- dès que cette opération est terminée, le microprocesseur 24 autorise le début d'une suite de séquences d'émission du codeur-émetteur radioélectrique 26 ; la chronologie de cette suite est fonction des caractéristiques du système spatial de collection de données utilisé; elle s'interrompt à l'épuisement de la batterie 18, et, si possible, avant l'éjection de la sonde suivante de la même base 1 (cette précaution a pour but de limiter la prolifération des émissions sur une même zone et de réduire le nombre de messages d'identification des sondes) ; chaque séquence, d'une durée maximum de quelques secondes, est commandée par l'électronique de la sonde à des intervalles de temps réguliers ; son début peut toutefois être retardé si, et aussi longtemps que, le circuit entre l'électrode 28 et la partie conductrice du corps 5 reste fermé, le support d'antenne 7 étant alors probablement noyé temporairement ; le codeur/émetteur radioélectrique 26 n'est mis sous tension que pendant le temps nécessaire à la transmission des données comprimées vers le satellite de collection de données, selon la procédure recommandée par son constructeur. Plusieurs séquences d'émission sont nécessaires pour assurer avec un niveau de probabilité convenable, la bonne réception par le satellite de la totalité du contenu des messages à transmettre. En exploitation normale, il n'est pas demandé à l'exploitant du système satellite de fournir les données de localisation de l'émission; ceci est néanmoins possible, en particulier avec le système ARGOS.
- les mesures recueillies par le satellite sont transmises au centre de traitement, qui réalise successivement les opérations suivantes :

### (a) restitution du profil de profondeur en fonction du temps D=f(t).

Pour reconstituer chaque profil de sondage avec précision, on dispose au centre de traitement d'un ensemble d'informations susceptible d'améliorer celle-ci, d'affecter un degré de confiance à la valeur et à la précision des résultats obtenus, ou encore de détecter des accidents de remontée; ces informations sont: les instants d'éjection et d'arrivée en surface, la pression au niveau de la plateforme mesurée par le capteur 13, la connaissance de la vitesse de remontée de la sonde obtenue à partir de mesures hydrodynamiques. Le profil le plus probable de D=f(t) s'obtient en intégrant la vitesse de remontée et en ajustant le résultat obtenu pour tenir compte des valeurs aux limites mesurées à l'éjection et à l'arrivée en surface. Dans cette intégration, il peut être important de tenir compte de divers facteurs correcteurs, dont les principaux sont la variation du poids d'eau déplacé par la sonde due à la compressibilité de ses matériaux, les variations de densité du milieu si on les connaît, les tolérances sur la pesée et le Cx des sondes.

### (b) restitution du (ou des) profil(s) Grandeur(s) mesurée(s)en fonction de la profondeur.

La relation entre une grandeur mesurée G et la profondeur D, G=f(D) est déterminée au Centre de traitement en rapprochant les relations G=f(t) déduites des enregistrements et D = f(t) obtenue par la méthode ci-dessus. Les temps t sont déterminés par la même horloge, ce qui constitue évidemment un facteur favorable à la sécurité et à la précision des mesures.
- le centre de traitement trie, diffuse et stocke les résultats obtenus, en fonction des demandes des utilisateurs. Il peut également offrir à ces derniers des produits élaborés à partir des résultats de traitement. Il convient de noter que la nature et la présentation des données brutes, ainsi que le court délai qui sépare les relevés de profils de l'arrivée des données au centre de traitement sont particulièrement adaptés à un fonctionnement automatique à vocation opérationnelle.

La description précédente se réfère à une utilisation en mer libre ; il est aussi possible d'utiliser le procédé et le dispositif suivant l'invention en mer polaire, à surface libre l'été et couverte en surface d'une banquise dérivante en hiver. Dans un tel cas d'utilisation :
- on immerge les bases d'observation pendant l'été, conformément au procédé selon l'invention,
- on charge les instructions du programmateur 12 de façon à commander la réalisation des profils verticaux à tout moment choisi par l'expérimentateur, au cours des douze mois suivants, conformément au procédé suivant l'invention,
- on charge les instructions du programmateur de sonde 20 pour réaliser des opérations d'acquisition des mesures en profil, traitement et mise en mémoire des données, conformément au procédé selon l'invention, et pour inhiber la commande de mise sous tension du codeur/émetteur radioélectrique 26 jusqu'à une date donnée (par exemple, le milieu de l'été suivant l'immersion de la station considérée).

Dans ce cas d'utilisation, on ne pourra donc immerger les stations en toutes saisons, ni recueillir en temps quasi-réel les résultats. Il n'en reste pas moins que le procédé répond à une demande importante des expérimentateurs, très mal satisfaite actuellement.

Des précautions de construction des sondes devront être prises pour éviter l'écrasement des sondes par la glace, au moment de sa formation ; si les matériaux utilisés dans la construction desdites sondes ne sont pas en mesure de résister aux pressions ainsi développées, on les revêtira d'une peau épaisse 2' en mousse élastique à cellules fermées.

Une première application de l'invention concerne la réalisation de séries chronologiques de profils de température et de conductivité d'une colonne d'eau marine en fonction de la profondeur. Les mesures de température sont réalisées directement en utilisant un capteur 16 de température à sonde de platine. Le capteur 17 de conductivité est constitué par un ensemble de quatre électrodes montées dans un circuit électronique; le principe de la mesure a été décrit par plusieurs auteurs (cf. par exemple le brevet des U.S.A. n°3.939.408). Ce type de capteur est particulièrement sensible aux agressions du milieu: la protection offerte par la capsule 9 élimine totalement cette difficulté; les possibilités offertes par l'invention pour étalonner et calibrer régulièrement les capteurs sont particulièrement adaptées à la détection de dérives des chaînes de mesure de conductivité.

Connaissant la température et la conductivité de l'eau de mer en fonction de la profondeur, on peut déterminer son profil de salinité (voir par exemple l'ouvrage de P. MOREL "Introduction à la dynamique de l'Atmosphère, de l'Océan et du Climat", page 17, éd. Institut Océanographique, Paris).

L'application à la mesure de séries chronologiques de température et de salinité de colonnes et de masses d'eau marines est susceptible de remplacer au moins en partie les systèmes décrits en (A), (B) et (C) ci-dessus, dans le domaine des sondages océaniques; elle devrait en particulier constituer un élément essentiel d'un système de surveillance de la circulation océanique à différentes échelles spatiales. Elle trouve également son application dans la réalisation de séries chronologiques de mesures en un point géographique donné, soit en remplacement de stations fixes par bouées ancrées, soit à la place de sondages réalisés régulièrement par navire spécialisé, soit enfin comme suite à des prélèvements hydrologiques par navire, en vue de suivre l'évolution dans le temps de la composition d'une colonne d'eau.

L'invention ne fait appel à aucune technologie nouvelle; elle repose au contraire sur la combinaison de solutions techniques éprouvées.

Selon l'objectif poursuivi (nature des grandeurs à mesurer, précision des mesures, probabilité de bon fonctionnement, durée de vie, prix unitaire), les choix de configuration et de matériaux de la sonde, peuvent sensiblement varier. A titre d'exemple, dans le cas de mesures de profils de température et conductivité comportant les objectifs de précision suivants: température ±0,05°C, conductivité ±0,25% de la gamme de mesure, profondeur ±15m pour une immersion maximum de 2000m, une durée de vie de trois ans et une probabilité de bon fonctionnement de 90%, on pourra construire la sonde en utilisant une électronique à composants grand public, placée dans un corps 5 de 25 mm de diamètre intérieur par exemple, réalisé partie (41) en fibre de verre bobinée, partie (40) en alliage de zinc renforcé par un fourreau en acier, assemblés par collage, des batteries lithium-soufre, une huile légère (densité 0,7) contenue dans une enveloppe en matière plastique comme matériau de flottabilité, une antenne hélicoïdale déposée sur la pièce porte-antenne 7, un capteur 17 de conductivité à électrodes disposées concentriquement deux à deux de manière diamétralement opposée sur deux côtés de la pièce porte-capteurs 8, et un capteur 16 de température par sonde Pt 100.

Les Fig. 5 et 6 présentent à titre d'exemple et de manière schématique, une réalisation possible du mécanisme d'éjection selon l'invention, répondant avantageusement aux besoins de celle-ci, à savoir longue durée de vie dans le milieu marin et faible coût: avant commande d'éjection, la sonde 2 repose par l'intermédiaire de ses trois empennages 30 sur une alvéole annulaire 45 du réceptacle 11. Un bras 35, rigide, articulé en 36, est rappellé en position de maintien de la sonde 2, par un câble 37 lui-même rattaché au tube central 1" de la base 1 par un brin 38 électrolysable rapidement dans l'eau de mer (par exemple un fil de magnésium), par application d'un potentiel électrique. Un levier 39 articulé en 46 est relié à une de ses extrémités au bras 35 par un câble 47 et à l'autre à un oeil 48 de la capsule 9 par un lien 49 mis en place au montage de la sonde 2 sur le réceptacle 11. Enfin, un sandow 44 est tendu pour rappeler le bras 35 et permettre son dégagement le long du tube central de la base 1. Le mécanisme fonctionne de la manière suivante : peu après application d'un potentiel électrique sur le brin 38, celui-ci se rompt: le sandow 44 rappelle le bras 35 ; par l'intermédiaire du câble 47 et du levier 39, la capsule 9 se détache du corps 5 de sonde; sa libération permet au bras 35 de s'effacer complètement pour laisser le champ libre à l'ascension de la sonde.

La structure de la base 1 peut être avantageusement conçue comme réticulée, autour d'une charpente centrale reprenant les efforts engendrés par le poids du lest pendant la descente, selon une conception semblable à celle des structures de prélèvement hydrologiques par bouteilles.

On peut citer, de manière non exhaustive, les avantages suivants du procédé et du dispositif selon l'invention, par rapport aux procédés et dispositifs utilisés actuellement: (1) réduction du coût d'acquisition des données océanographiques, surtout lorsque l'expérimentation a pour but une surveillance opérationnelle nécessitant de nombreuses saisies répétitives de mesures réparties sur plusieurs sites et une longue période de temps, (2) plus grande flexibilité dans le choix des emplacements géographiques et des calendriers de sondage, permettant en particulier d'obtenir des vues synoptiques par "coupes" horizontales et verticales des masses d'eau, ou encore de "phaser" des observations par rapport à des phénomènes périodiques comme la saison ou la marée, (3) plus grandes répétitivité et comparabilité des conditions de mesure, facteur de qualité des résultats, (4) meilleure protection des capteurs, permettant d'éliminer toute cause de dégradation due à l'environnement marin, (5) importante réduction des délais, coûts et aléas de transmission, de traitement et de diffusion des données, rendue possible par l'automatisation complète de la chaîne de collecte et de traitement, (6) possibilité de réserver une haute confidentialité au processus, depuis la mise en place des stations fond de mer jusqu'à la diffusion des données, permettant une exploitation classifiée et/ou commerciale du système de collecte de données par son propriétaire; s'adapte particulièrement bien à une gestion efficace et peu coûteuse du traitement des données et à la diffusion de produits élaborés à des utilisateurs aux besoins variés.

Bien entendu, l'invention n'est pas limitée à la mesure de la température et de la conductivité de l'eau de mer en fonction de la profondeur. Elle trouve également son application au profilage de toute grandeur caractéristique du milieu marin, à partir de tout capteur susceptible de caractériser ladite grandeur et d'être réalisé sous une forme susceptible d'être embarqué sur un dispositif de sondage tel que décrit ci-dessus. C'est ainsi que paraissent applicables le profilage direct en densité de l'eau de mer à partir de réflectomètres optiques, en vitesse du son par vélocimètres acoustiques, en répartition de colonies planctoniques par mesures optiques de fluorescence, etc...

## Revendications

1. Procédé pour réaliser des profils verticaux de la mesure d'au moins une grandeur caractéristique d'une masse d'eau marine, à l'aide d'au moins une sonde (2) plus légère que l'eau et équipée d'un capteur de mesure de ladite grandeur, caractérisé en ce que :
- on coule ladite sonde (2) sur le fond marin par lestage,
- on commande la remontée de la sonde (2) et l'activation du capteur pour l'enregistrement de la mesure de ladite grandeur au long de la colonne d'eau traversée par la sonde (2) jusqu'à la surface de la masse d'eau, et
- on commande une transmission des mesures relevées vers un centre d'exploitation de ces mesures.

2. Procédé conforme à la revendication 1, caractérisé en ce qu'on coule une pluralité de sondes (2) sur le fond marin, et en ce qu'on commande séquentiellement la remontée de chacune des sondes.

3. Procédé conforme à la revendication 2, caractérisé en ce qu'on coule plusieurs groupes de sondes (2) en des sites prédéterminés du fond marin, et en ce qu'on commande la remontée coordonnée d'au moins une pluralité de sondes (2) provenant de groupes différents.

4. Procédé conforme à l'une quelconque des revendications 1 à 3, caractérisé en ce que, pour restituer le profil de mesure de la grandeur caractéristique de la masse d'eau en fonction de la profondeur, on rapproche par coïncidence dans le temps les données obtenues par (a) la mesure de ladite grandeur réalisées à des instants (t), datés par une horloge (19) de bord de la sonde, (b) la reconstitution de la relation donnant la profondeur de la sonde en fonction du temps (t) relevé par la même horloge, à partir de la connaissance de la vitesse de remontée de la sonde (2), d'une part, et des mesures de pression et de temps de départ et d'arrivée en surface de la sonde (2), d'autre part.

5. Procédé conforme à l'une quelconque des revendications 1 à 4, caractérisé en ce qu'on mesure deux grandeurs caractéristiques de la masse d'eau à l'aide de deux capteurs (16) et (17) de température et de conductivité respectivement, en vue de réaliser des profils verticaux de mesure de température et de salinité de ladite masse d'eau en fonction de la profondeur.

6. Procédé conforme à la revendication 5, caractérisé en ce que :
- on étalonne les chaînes de mesure de température et de conductivité de chaque sonde, en fin de construction en usine, par immersion des capteurs (16) et (17) dans une succession de bains étalonnés en température et salinité,
- on immerge ensuite les capteurs (16) et (17) dans un liquide étalonné en salinité, dans lequel ils restent confinés pendant les phases de stockage en atelier et sur le fond marin, jusqu'à éjection de la sonde (2) de sa base (1),
- on réalise des calibrations des chaînes de mesure à intervalles réguliers, par référence au liquide étalon, de manière automatique, l'activation de ces calibrations étant commandée par l'horloge (19) de la sonde (2), et
- on enregistre les mesures de calibration dans la sonde (2), pour restitution ultérieure dans le centre d'exploitation des mesures enregistrées par la sonde.

7. Dispositif pour la mise en oeuvre du procédé conforme à la revendication 1, caractérisé en ce qu'il comprend :
- au moins une sonde (2) munie d'au moins un capteur (16,17) de mesure d'au moins une grandeur à relever, de moyens (18 à 26) d'acquisition, de stockage et de transmission des mesures réalisées et de moyens (6) pour lui donner une flottabilité positive, et
- une base (1) lestée pour reposer sur le fond marin et équipée de moyens (35) pour retenir temporairement ladite sonde (2) et libérer celle-ci à un instant prédéterminé.

8. Dispositif conforme à la revendication 7, caractérisé en ce que plusieurs sondes (2) sont montées sur la base (1).

9. Dispositif conforme à l'une quelconque des revendications 7 ou 8, caractérisé en ce que la base (1) comprend des moyens de programmation (12) et de commande (38) du largage des sondes (2) et une source d'énergie électrique (14) alimentant ces moyens.

10. Dispositif conforme à l'une quelconque des revendications 7 à 9, caractérisé en ce que chaque sonde (2) est équipée de moyens (40,43) pour couler cette sonde après la transmission des mesures relevées.

11. Dispositif conforme à la revendication 10, caractérisé en ce que lesdits moyens (40,43) assurent une destruction progressive par électrolyse d'un élément de paroi de la sonde (2) après le largage de la sonde (2).

12. Dispositif conforme à la revendication 7, pour la mise en oeuvre du procédé selon la revendication 6, caractérisé en ce que les capteurs de température (16) et de conductivité (17) sont protégés par une capsule (9) remplie de liquide étalon, emboîtée sur le corps (5) de la sonde (2), et montée de manière à se détacher de celui-ci lors de l'activation des moyens (35) de libération de la sonde.

13. Dispositif conforme à la revendication 7, pour la mise en oeuvre du procédé selon revendication 5, caractérisé en ce que la base (1) porte au moins un capteur de pression (13) et des moyens pour transmettre la mesure correspondante jusqu'au centre d'exploitation des mesures.

14. Sonde formant partie du dispositif conforme à l'une quelconque des revendications 7 à 13.

15. Sonde conforme à la revendication 14, caractérisée en ce qu'elle comprend une enveloppe (2') en un matériau déformable propre à absorber la pression subie par la sonde lors d'une prise dans la glace.

16. Utilisation des mesures délivrées par le dispositif conforme à l'une quelconque des revendications 7 à 13, pour la fourniture de séries chronologiques de profils verticaux de mesures de grandeurs représentatives du milieu marin.

## Patentansprüche

1. Verfahren zum Erzeugen von vertikalen Meßprofilen wenigstens einer meerwassercharakteristischen Größe unter Mitwirkung wenigstens einer solchen Sonde (2), die leichter als Wasser ist und die mit einem Meßwertaufnehmer für die genannte Größe ausgerüstet ist,
dadurch gekennzeichnet,
- daß die genannte Sonde (2) mittels eines Ballastes bis auf den Meeresboden abgesenkt wird,
- daß der Wiederaufstieg der Sonde (2) und die Aktivierung des Meßwertaufnehmers zum Zweck des Registrierens der genannten Meßgröße entlang der, durch die Sonde (2) bis zum Erreichen der Wasseroberfläche durchquerten Wassersäule gesteuert wird, und
- daß die Übertragung der aufgenommenen Meßwerte in Richtung auf ein Auswertungszentrum für diese Meßwerte hin gesteuert wird.

2. Verfahren nach Anspruch 1,
dadurch gekennzeichnet,
- daß eine Vielzahl von Sonden (2) auf den Meeresboden abgesenkt wird und
- daß der Wiederaufstieg einer jeden der Sonden aufeinanderfolgend gesteuert wird.

3. Verfahren nach Anspruch 2,
dadurch gekennzeichnet,
- daß mehrere Gruppen von Sonden (2) an vorherbestimmten Stellen auf den Meeresboden abgesenkt werden und
- daß der koordinierte Wiederaufstieg wenigstens einer Vielzahl an aus unterschiedlichen Gruppen herrührenden Sonden gesteuert wird.

4. Verfahren nach einem der vorangegangenen Ansprüche 1 bis 3,
dadurch gekennzeichnet,
- daß zum Zweck der Gewinnung des Meßwertprofils der wassercharakteristischen Größe in Abhängigkeit von der Tiefe die gewonnenen Daten, die dadurch erhalten worden ist, daß (a) die genannte Meßgröße zu den Zeitpunkten (t) erzeugt worden ist, die mittels einer sich in der Sonde befindenden Uhr (19) datiert worden sind und daß (b) die Funktion, welche die Tiefe der Sonde in Abhängigkeit von der Zeit (t) beschreibt, mittels der gleichen Uhr aufgenommen worden ist, ausgehend von der Kenntnis der Geschwindigkeit des Wiederaufstiegs der Sonde (2) einerseits, von Druckmessungen und von Messungen der Zeitpunkte des Beginns des Aufstiegs der Sonde (2) und deren Ankunft an der Oberfläche andererseits einander zeitlich zugeordnet werden.

5. Verfahren nach einem der vorangegangenen Ansprüche 1 bis 4,
dadurch gekennzeichnet,
- daß zwei wassercharakteristische Größen mittels zweier, jeweils zur Erfassung der Temperatur und der Leitfähigkeit bestimmter Meßwertaufnehmer (16, 17) gemessen werden, um vertikale, die Temperatur und den Salzgehalt des Wassers in Abhängigkeit von der Tiefe darstellende Meßprofile zu erzeugen.

6. Verfahren nach Anspruch 5,
dadurch gekennzeichnet,
- daß die, die Temperatur und die Leitfähigkeit betreffenden Meßwertketten einer jeden Sonde am Ende der herstellerseitigen Montage dadurch geeicht werden, daß die Aufnehmer (16, 17) in eine Reihe von hinsichtlich der Temperatur und des Salzgehaltes geeichten Bädern eingetaucht werden,
- daß die Aufnehmer (16, 17) anschließend in eine hinsichtlich des Salzgehaltes geeichte Flüssigkeit eingetaucht werden, in der sie während der Phasen der herstellerseitigen Lagerung und der Lagerung auf dem Meeresboden eingeschlossen bleiben und zwar bis zum Ausstoß der Sonde (2) aus ihrem Grundkörper (1),
- daß die Einstellungen der Meßwertketten nach Maßgabe gleichförmiger Zeitintervalle in automatischer Weise unter Bezugnahme auf die Eichflüssigkeit vorgenommen werden, wobei diese Eichungen mittels der Uhr (19) der Sonde (2) gesteuert werden und
- daß die Eichmeßwerte in der Sonde (2) registriert werden, um diese später in dem Auswertungszentrum für die mittels der Sonde registrierten Meßwerte erneut zu gewinnen.

7. Vorrichtung zur Durchführung des Verfahrens nach Anspruch 1,
gekennzeichnet durch
- wenigstens eine, mit wenigstens einem Meßwertaufnehmer (16, 17) für eine aufzunehmende Größe ausgerüstete Sonde (2), mit Mitteln (18 bis 26) zur Erfassung, zur Speicherung und zur Übertragung der erzeugten Meßwerte und mit Mitteln (6), um ihr einen positiven Auftrieb zu verleihen und
- mit einem Grundkörper (1), der mit einem Ballast versehen und zur Aufstellung auf dem Meeresboden bestimmt ist und der mit Mitteln (35) ausgerüstet ist, um die genannte Sonde (2) vorübergehend zurückzuhalten und um diese zu einem vorherbestimmten Zeitpunkt freizugeben.

8. Vorrichtung nach Anspruch 7,
dadurch gekennzeichnet,
- daß auf dem Grundkörper (1) mehrere Sonden (2) angeordnet sind.

9. Vorrichtung nach einem der Ansprüche 7 oder 8,
dadurch gekennzeichnet,
- daß der Grundkörper (1) Mittel zur Programmierung (12) und zur Steuerung (38) der Freigabe der Sonden (2) sowie eine, diese Mittel versorgende elektrische Energiequelle (14) aufweist.

10. Vorrichtung nach einem der Ansprüche 7 bis 9,
dadurch gekennzeichnet,
- daß eine jede Sonde (2) mit Mitteln (40, 43) zum Absenken dieser Sonde nach erfolgter Übertragung der aufgenommenen Meßwerte ausgerüstet ist.

11. Vorrichtung nach Anspruch 10,
dadurch gekennzeichnet,
- daß die genannten Mittel (40, 43) eine fortschreitende elektrolytische Zerstörung eines Wandungselements (2) der Sonde nach erfolgter Freisetzung der Sonde (2) sicherstellen.

12. Vorrichtung gemäß Anspruch 7 zur Durchführung des Verfahrens gemäß Anspruch 6,
dadurch gekennzeichnet,
- daß die Aufnehmer (16) für die Temperatur und (17) für die Leitfähigkeit durch eine Kapsel (9) geschützt angeordnet sind, die mit einer Eichflüssigkeit ausgefüllt ist, die an den Körper (5) der Sonde (2) angeschlossen ist, und die derart angeordnet ist, daß sie sich infolge der Aktivierung der Mittel (35) zur Freigabe der Sonde von dem Körper löst.

13. Vorrichtung gemäß Anspruch 7 für die Durchführung des Verfahrens gemäß Anspruch 5,
dadurch gekennzeichnet,
- daß der Grundkörper (1) wenigstens einen Aufnehmer (13) für den Druck und Mittel zur Übertragung des entsprechenden Meßwertes zu dem Auswertungszentrum für Meßwerte umfaßt.

14. Sonde, die einen Teil der Vorrichtung gemäß einem der Ansprüche 7 bis 13 bildet.

15. Sonde nach Anspruch 14,
dadurch gekennzeichnet,
- daß sie einen, aus einem verformbaren Werkstoff bestehenden Hüllkörper (2') aufweist, der dazu geeignet ist, einen durch die Sonde aufzunehmenden Druck infolge eines Einschlusses in Eis zu absorbieren.

16. Verwendung der mittels der Vorrichtung nach einem der vorangegangenen Ansprüche 7 bis 13 gelieferten Meßwerte zur Erzeugung chronologischer Folgen vertikaler Meßprofile solcher Größen, die für das Meeresmilieu repräsentativ sind.

## Claims

1. Method of producing vertical profiles of at least one measured variable characterising a body of sea water using at least one lighter-than-water probe (2) equipped with a sensor for measuring said variable, characterised in that:
- said probe (2) is dropped by means of a ballast to the sea bed,
- the ascent of the probe (2) and activation of the sensor are commanded in order to record the measured value of said variable along the column of water through which the probe (2) passes before reaching the surface of the body of water, and
- transmission of the resulting measured values to a measurement processing centre is commanded.

2. Method according to claim 1 characterised in that a plurality of sensors (2) are dropped to the sea bed and in that the ascent of each probe is commanded sequentially.

3. Method according to claim 2 characterised in that a plurality of groups of probes (2) are dropped at predetermined sites on the sea bed and in that the coordinated ascent of at least one plurality of probes (2) from different groups is commanded.

4. Method according to any one of claims 1 to 3 characterised in that, in order to output the measured value profile of the variable characterising the body of water as a function of depth, data obtained (a) by the measurement of said variable at times (t) dated and timed by a clock (19) on board the probe and (b) by reconstitution of the relation giving the depths of the probe as a function of the time (t) given the same clock from the known rate of ascent of the probe (2), on the one hand, and measurements of pressure and of the starting time and arrival at the surface time of the probe (2), on the other hand, is combined.

5. Method according to any one of claims 1 to 4 characterised in that two variables characterising the body of water are measured by means of two sensors (16) and (17), respectively measuring temperature and conductivity, in order to produce vertical measurement profiles of the temperature and of salinity of said body of water as a function of depth.

6. Method according to claim 5 characterised in that:
- the temperature and conductivity measuring systems of each probe are calibrated at the end of construction in the factory by immersing the sensors (16) and (17) in a succession of baths at standard temperature and salinity,
- the sensors (16) and (17) are then immersed in a liquid with standard salinity in which they remain during storage in the workshop and on the sea bed, until ejection of the probe (2) from its base (1),
- the measuring systems are automatically calibrated at regular intervals, with reference to the standard liquid, activation of such calibration being commanded by the clock (19) of the probe (2), and
- the calibration measurements are recorded in the probe (2) for subsequent output in the processing centre of measurements recorded by the probe.

7. Device for implementing the method according to claim 1 characterised in that it comprises:
- at least one probe (2) equipped with at least one sensor (16, 17) for measuring at least one variable, means (18 through 26) for acquiring, storing and transmitting the resulting measured values and means (6) for conferring positive buoyancy on it, and
- a base (1) ballasted to remain on the sea bed and equipped with means (35) for temporarily retaining said probe (2) and releasing it at a predetermined time.

8. Device according to claim 7 characterised in that a plurality of probes (2) are mounted on the base (1).

9. Device according to claim 7 or claim 8 characterised in that the base (1) comprises programming means (12) and control means (38) for releasing the probes (2) and an electrical power supply (14) supplying power to said means.

10. Device according to any one of claims 7 to 9 characterised in that each probe (2) is equipped with means (40, 43) for sinking the probe after transmission of the measured values.

11. Device according to claim 10 characterised in that said means (40, 43) assure progressive destruction by electrolysis of a wall element of the probe (2) after the probe (2) is released.

12. Device according to claim 7 for implementing the method according to claim 6 characterised in that the temperature sensor (16) and the conductivity sensor (17) are protected by a capsule (9) filled with standard liquid, nested over the body (5) of the probe (2) and mounted so as to separate from the latter on activation of the means (35) for releasing the probe.

13. Device according to claim 7 for implementing the method according to claim 5 characterised in that the base (1) carries at least one pressure sensor (13) and means for transmitting the corresponding measured value to the measurement processing centre.

14. Probe forming part of the device according to any one of claims 7 to 13.

15. Probe according to claim 14 characterised in that it comprises a deformable material jacket (2') adapted to absorb pressure exerted on the probe when trapped in ice.

16. Use of measurements supplied by the device according to any one of claims 7 to 13 to supply chronological series of vertical profiles of measured variables characterising the marine environment.
